# EUROPEAN PATENT APPLICATION

(11) **EP 3 735 945 A1**
(43) Date of publication of application: **11.11.2020**
(21) Application number: 19902687.3
(22) Date of filing: 28.06.2019
(51) Int. Cl.: A61F 5/02

(54) **POSTURE CORRECTOR**

(30) Priority: 28.12.2018 RU 2018147327
(71) Applicant: AKTSIONERNOE OBSHCHESTVO "TONUS", Bryanskaya obl., 243300 (RU)
(72) Inventor: KHASIN, Igor Leonidovich, 2167 Marure (LV); NOZDRYA, Ekaterina Mikhajlovna, Unecha, Bryanskaya obl., 243300 (RU); NATALUKHA, Sergej Viktorovich, Unecha, Bryanskaya obl. 243300 (RU)
(74) Representative: Vitina, Maruta
(86) International application number: PCT/RU2019/050098
(87) International publication number: WO 2020/139166

(57) **Abstract**

The posture corrector is an orthopedic corset and is formed from a single ribbon made of an elastic fabric having an extensibility of 70-100 % and density of 420-510 N/m². The ribbon comprises: a first section, which is a first free end of the ribbon; a second section, which is a first loop formed by two subsequent bends of the ribbon; a third section, which is a second loop formed by two subsequent bends of the ribbon; a fourth section, which is an intersection of the beginning of the first loop and the end of the second loop; a fifth section, which is a second free end of the ribbon. The fourth section is fixed. The first and the second loops form the shoulder straps. The free ends of the ribbon are made with the possibility of connecting with each other on the front of the user, thus forming a belt. The invention increases the comfort of wearing a posture corrector.

## Description

The invention relates to an orthopedic medical device, namely an orthopedic corset, intended for the prevention and rehabilitation of various diseases associated with spinal deformity.

There is known posture corrector in the art, which is made of an elastic material and composed of a vertically mounted spinal support with shoulder straps diverging from its top and provided with fastening elements at the ends. The straps are adjustable in length by means of buckles and are provided with several successively located locking elements at the ends (RU 66672 U1 27.09.2007).

There is also known a posture corrector that has a first strap forming its first loop, a first free end of the strap and a first fastening point; and a second strap forming a second loop and a second fastening point, wherein the first loop is placed on one shoulder of the user, and the second loop is placed on the other shoulder of the user. The free ends of the first and second straps pass from back to front of the patient's body, wrapping him. Then, the first fastening point and the second fastening point are connected on the front side of the user's body. The straps of the posture corrector are made of a combination of elastic, semi-elastic and rigid materials. (US20150105710 A1 16.04.2015).

Along with the foregoing, a posture corrector is known from the prior art, which is a fabric strap with central longitudinal slots. The free ends of the strap are crossed and passed through the slots, forming two loops for the user's arms. Additionally, the free ends of the strap have fasteners in the form of a buckle to connect the opposite ends after tightening the strap, thereby trying to pull back the shoulders of the user and straighten the back of the user (US 20030153855 A1 14.08.2003).

The disadvantage of all previously known posture correctors is the lack of wearing comfort, since they are made with rigid elements, wire, or buckles; the shoulder straps are made in such a way that either exert too much pressure on the soft tissues, or do not exert the necessary pressure.

The aim of the present invention is to increase the comfort of wearing a posture corrector, due to the absence of rigid inserts, wire, or fastening belts; and the design of the arm straps that ensures uniform distribution of load on the spine due to the material from which the posture corrector is made.

The technical result of the present invention is to increase the comfort of wearing a posture corrector.

The technical result is implemented due to the following design features. The present posture corrector is made of ribbon consisting of an elastic fabric that does not have any rigid elements, has an extensibility of 70-100% and a surface density of 420-510 N/m², which creates the necessary compression and provides convenient wearing of the posture corrector.

The posture corrector is formed of a single ribbon, which is made of an elastic fabric having an extensibility of 70-100% and a density of 420-510 N/m², and has: a first section (1), which is a first free end of the ribbon; a second section (2), which is a first loop formed by two (3 and 4) subsequent bends of the ribbon; a third section (5), which is a second loop formed by two subsequent bends of the ribbon (6 and 7); a fourth section, which is an intersection of the beginning of the first loop and the end of the second loop (8); a fifth section (9), which is a second free end of the ribbon. The fourth section, formed by the intersection of the beginning of the first loop and the end of the second loop, is fixed. The first and the second loops form a shoulder straps. A loop tape is fixed on one of the free ends of the ribbon on the outer side. On the other free end of the ribbon, respectively, on the inner side, is a hook tape. The free ends of the ribbon are made with the possibility of connecting with each other on the front of the user, thus forming a belt.

In addition, on the loops in the area of contact with the user's skin a softening pad is placed. Thus, the use of an elastic fabric ribbon that does not have any rigid elements, having extensibility of 70-100% and density of 420-510 N/m², the design features of the posture corrector: the division of the ribbon into sections when forming the straps and the belt, as well as the use of softening pads on the straps, the lack of contact of the fasteners with the patient's skin, allows to increase the wearing comfort of the posture corrector. Since there are no issues like: rubbing the skin with the fastener causing irritation at the points of contact; restricting the user's movements (absence of hard elements), pulling shoulders and arms (softening pads). Therefore, the plurality of these design features implements the abovementioned technical result, which consists in increasing the comfort of wearing the posture corrector.

In addition, the length of the ribbon provides the size of the corrector and depends on the chest circumference level of the user's celiac plexus. Moreover, in turn, the size of its sections depends on the size of the corrector - the length of the ribbon. Sizes are selected according to the values shown in Tables 1-4.

An example of formation of the corrector:
The seamstress takes a cut piece of fabric for the future posture corrector - a ribbon of a certain length depending on the chest circumference at the user's celiac plexus level. She carries out a visual inspection of its cutting for the presence of defects: missed stitches, dirty oil stains, or holes. The front side of the posture corrector (the ribbon) is the side of the fabric with visible mesh of polyester threads.

The seamstress makes the necessary markings on the fabric to mark the sections of the ribbon. Then she lays the loops on the inner side to form the straps, in accordance with the marking. She adjusts the softening pad with a zigzag stitch; trims the edge of the fabric (free ends of the ribbon) - with a 1.7 cm wide trimming band using a single-needle lockstitch sewing machine with a straight-stitch overlocker. On the first free end of the ribbon from the front side, she sews the loop ribbon with a zigzag stitch, while fixing the ends of the trimming band and attaching fabric labels. A hook tape is sewn onto the second free end of the ribbon from the inner side with a zigzag stitch, while simultaneously securing the ends of the trimming band. The ribbon folded in two loops with the face side up is attached to the central part. At that, the distance from the lower edge of the obtained intersection to the end of both free ends should be the same and equal to L₁. Then the intersection of the loops and the central part is fixed with a zigzag stitch along the intersection contour. The excess threads and trimming band are cut off.

Example 1. Description of the process of tailoring a posture corrector of size 3. To make a posture corrector, take a 6 cm wide ribbon with a density of 495 g/m² and extensibility of 97%. At the cutting site, the ribbon is inspected from the outer and the inner side for the presence of defects. Then, using a pattern, cut out a posture corrector of size 3 (the length of the product in the cut is 205 cm). At the same time, make the necessary marks on the fabric. Using an additional pattern, cut out the softening pad (for the 3rd size, its length is 27 cm and its width is 6 cm). After that, the cut details arrive at the sewing area. Before starting work, the seamstress checks the completeness of the cut details and the presence of defects. Then she places the softening pad face up on the inner side of the cut-out posture corrector, in accordance with the marking and at a distance of 1 cm from the edge of the ribbon, and fixes it from three sides using a single-needle lockstitch sewing machine with a zig-zag stitch. The edge of the fabric along with the softening pad is folded towards the outer side of the product, and the margin of the softening pad is fixed to the fabric with zig-zag stitch, thus forming a roller. The edges of the fabric are trimmed using a single-needle lockstitch sewing machine with a straight-stitch overlocker. For trimming, use a 1.7 cm wide trimming band. On one edge of the fabric, from its outer side, the seamstress places a 15 cm long loop tape and stitches it along the perimeter while fixing the ends of the trimming band. While stitching, she puts under the loop tape the fabric labels indicating the size of the product and the composition of the raw material. On the other edge of the fabric, from its inner side, she places the hook tape and stitches it along the perimeter with a zig-zag stitch while fixing the ends of the trimming band. She folds the fabric in the shape of an "8"; for the 3^{rd} size, the distance from the edge of the product to the lower edge of the intersection is 39 cm. The rollers on the straps should be inside the "8". She aligns the O point with the lower corner of the rhombus obtained when crossing the straps, and stitches using a single-needle lockstitch sewing machine with a zig-zag stitch along the intersection of all the ribbons (the stitch also has the shape of a rhombus). She cuts off the excess threads, folds the product and passes it to the packaging site.

Example 2. Description of the process of tailoring a posture corrector of size 1. To make a posture corrector, take a 6 cm wide ribbon with a density of 460 g/m² and extensibility of 80 %. At the cutting site, the ribbon is inspected from the outer and the inner side for the presence of defects. Then, using a pattern, cut out a posture corrector of size 1 (the length of the product in the cut is 175 cm). At the same time, make the necessary marks on the fabric. Using an additional pattern, cut out the softening pad (for the 1^{st} size, its lengths is 25 cm, and its width is 6 cm). After that, the cut details arrive at the sewing area. Before starting work, the seamstress checks the completeness of the cut details and the presence of defects. Then she places the softening pad face up on the inner side of the cut-out posture corrector, in accordance with the marking and at a distance of 1 cm from the edge of the ribbon, and fixes it from three sides using a single-needle lockstitch sewing machine with a zig-zag stitch. The edge of the fabric along with the softening pad is folded towards the outer side of the product, and the margin of the softening pad is fixed to the fabric with zig-zag stitch, thus forming a roller. The edges of the fabric are trimmed using a single-needle lockstitch sewing machine with a straight-stitch overlocker. For trimming, use a 1.7 cm wide trimming band. On one edge of the fabric, from its outer side, the seamstress places a 15 cm long loop tape and stitches it along the perimeter while fixing the ends of the trimming band. While stitching, she puts under the loop tape the fabric labels indicating the size of the product and the composition of the raw material. On the other edge of the fabric, from its inner side, she places the hook tape and stitches it along the perimeter with a zig-zag stitch while fixing the ends of the trimming band. She folds the fabric in the shape of an "8"; for the 1^{st} size, the distance from the edge of the product to the lower edge of the intersection is 31 cm. The rollers on the straps should be inside the "8". She aligns the O point (position 8) with the lower corner of the rhombus obtained when crossing the straps, and stitches using a single-needle lockstitch sewing machine with a zig-zag stitch along the intersection of all the ribbons (the stitch also has the shape of a rhombus). She cuts off the excess threads, folds the product and passes it to the packaging site.

Example 3. Description of the process of tailoring a posture corrector of size 6. To make a posture corrector, take a 6 cm wide ribbon with a density of 510 g/m² and extensibility of 100 %. At the cutting site, the ribbon is inspected from the outer and the inner side for the presence of defects. Then, using a pattern, cut out a posture corrector of size 6 (the length of the product in the cut is 250 cm). At the same time, make the necessary marks on the fabric. Using an additional pattern, cut out the softening pad (for the 6^{th} size, its lengths is 29 cm, and its width is 6 cm). After that, the cut details arrive at the sewing area. Before starting work, the seamstress checks the completeness of the cut details and the presence of defects. Then she places the softening pad face up on the inner side of the cut-out posture corrector, in accordance with the marking and at a distance of 1 cm from the edge of the ribbon, and fixes it from three sides using a single-needle lockstitch sewing machine with a zig-zag stitch. The edge of the fabric along with the softening pad is folded towards the outer side of the product, and the margin of the softening pad is fixed to the fabric with zig-zag stitch, thus forming a roller. The edges of the fabric are trimmed using a single-needle lockstitch sewing machine with a straight-stitch overlocker. For trimming, use a 1.7 cm wide trimming band. On one edge of the fabric, from its outer side, the seamstress places a 15 cm long loop tape and stitches it along the perimeter while fixing the ends of the trimming band. While stitching, she puts under the loop tape the fabric labels indicating the size of the product and the composition of the raw material. On the other edge of the fabric, from its inner side, she places the hook tape and stitches it along the perimeter with a zig-zag stitch while fixing the ends of the trimming band. She folds the fabric in the shape of an "8"; for the 6^{th} size, the distance from the edge of the product to the lower edge of the intersection is 51 cm. The rollers on the straps should be inside the "8". She aligns the O point (position 8) with the lower corner of the rhombus obtained when crossing the straps, and stitches using a single-needle lockstitch sewing machine with a zig-zag stitch along the intersection of all the ribbons (the stitch also has the shape of a rhombus). She cuts off the excess threads, folds the product and passes it to the packaging site.

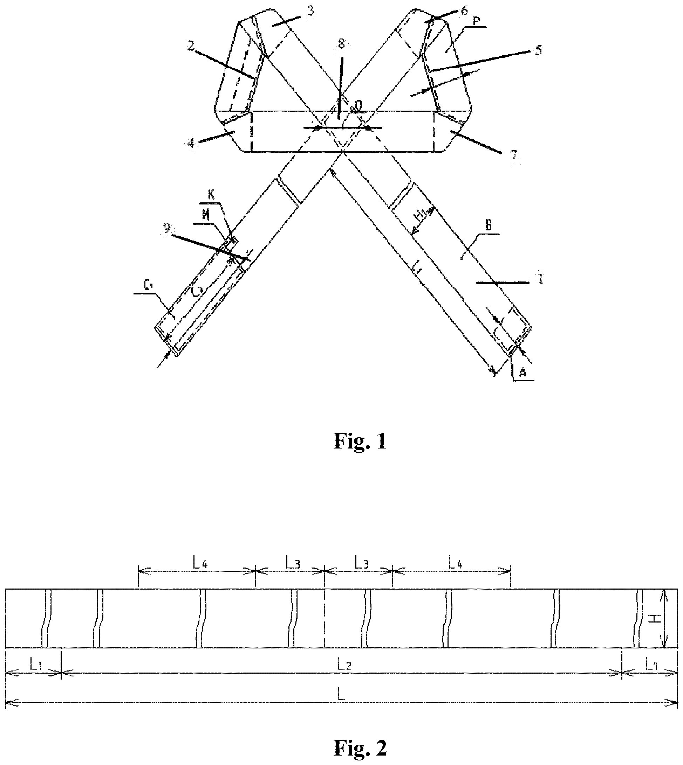

The posture corrector is illustrated in Figure 1 (view of the formed corrector) and Figure 2 (ribbon with marks from which the posture corrector is formed), which show: 1 - the first section of the ribbon, which is the first free end; 2 - the second section of the ribbon, which is the first loop; 3 - the first bend of the ribbon of the first loop; 4 - the second bend of the ribbon of the first loop; 5 - the third section of the ribbon, which is the second loop; 6 - the first bend of the second loop of the ribbon; 7 - the second bend of the second loop of the ribbon; 8 - the fourth section of the ribbon, which is the intersection of the beginning of the first loop and the end of the second loop; 9 - the fifth section, which is the second free end of the ribbon. **B** - elastic fabric, 6 cm wide (outer side); **L** - the length of the cut-out product, cm; **L₁** - product length from cut to intersection, cm; **O** - the middle of the part in section **L₂; L₃ -** the distance from the middle to the place of stitching of the softening pad, cm; **L₄ -** the length of the softening pad; **A** - trimming band, 1.7±0.2 cm wide; **A₁ -** trimming width, 0.6±0.1 cm; **C** - hook tape, 5 cm wide (H - the length of the hook tape, cm); **C₁** - loop tape, 5 cm wide; **C₂** - the length of the loop tape: 5 cm; **M** - fabric label indicating the size of the product; **K** - fabric label indicating the composition of the raw material of the product; **P-** softening pad.

**TABLE 1. LINEAR DIMENSIONS OF THE POSTURE CORRECTOR**

| Size | 1 | 2 | 3 | 4 | 5 | 6 | Tolerance |
|---|---|---|---|---|---|---|---|
| L, cm | 175 | 190 | 205 | 220 | 235 | 250 | ±5.0 |
| L₁, cm | 31 | 35 | 39 | 43 | 47 | 51 | ±2.0 |
| L₃, cm | 15 | 16 | 17 | 18 | 19 | 19 | ±1.5 |
| L₄, cm | 25 | 26 | 27 | 28 | 29 | 29 | ±1.5 |
| H, cm | 6 | 6 | 6 | 6 | 6 | 6 | ±0.5 |
| C₂, cm | 15 | 15 | 15 | 15 | 15 | 15 | ±1.0 |

**TABLE 2. LINEAR DIMENSIONS OF POSTURE CORRECTORS FOR CHILDREN AND ADOLESCENTS**

| Size | ID | 2D | 3D | Tolerance |
|---|---|---|---|---|
| L, cm | 140 | 152 | 165 | ±5.0 |
| L₁, cm | 23 | 26 | 29 | ±2.0 |
| L₃, cm | 12 | 13 | 14 | ±1.5 |
| L₄, cm | 22 | 23 | 24 | ±1.5 |
| H, cm | 6 | 6 | 6 | ±0.5 |
| C₂, cm | 10 | 10 | 15 | ±1.0 |

**TABLE 3. SELECTION OF POSTURE CORRECTOR SIZE**

| Size | 1 | 2 | 3 | 4 | 5 | 6 |
|---|---|---|---|---|---|---|
| Chest circumference at the celiac plexus level | 70-79 | 80-89 | 90-99 | 100-109 | 110-119 | 120-127 |

**TABLE 4. SELECTION OF POSTURE CORRECTOR SIZE FOR CHILDREN AND ADOLESCENTS**

| Size | 1 | 2 | 3 |
|---|---|---|---|
| Chest circumference at the celiac plexus level | 54-58 | 59-64 | 65-69 |

## Claims

1. A posture corrector formed from a single ribbon, **characterized in that** the ribbon is made of an elastic fabric having an extensibility of 70-100 % and density of 420-510 N/m² and has: a first section, which is a first free end of the ribbon; a second section, which is a first loop formed by two subsequent bends of the ribbon; a third section, which is a second loop formed by two subsequent bends of the ribbon; a fourth section, which is an intersection of the beginning of the first loop and the end of the second loop; a fifth section, which is a second free end of the ribbon; wherein the fourth section, formed by the intersection of the beginning of the first loop and the end of the second loop, is fixed; wherein the first and the second loops form a shoulder straps; a loop tape is fixed on one of the free ends of the ribbon on the outer side; on the other free end of the ribbon, respectively, on the inner side, is fixed a hook tape; the free ends of the ribbon are made with the possibility of connecting with each other on the front of the user forming a belt.

2. The posture corrector according to claim 1, **characterized in that** on the loops in the area of contact with the user's skin a softening pad is placed.

3. The posture corrector according to claim 1, **characterized in that** the length of the ribbon provides the size of the corrector and depends on the chest circumference level of the user's celiac plexus.
